# EUROPEAN PATENT APPLICATION

(11) **EP 4 625 432 A1**
(43) Date of publication of application: **01.10.2025**
(21) Application number: 24167871.3
(22) Date of filing: 29.03.2024
(51) Int. Cl.: G16H 40/20, G16H 40/40, G16H 40/63, A61B 6/00, A61B 5/02

(54) **AUTOMATED INSTRUCTION ENGINE**

(71) Applicant: Koninklijke Philips N.V., 5656 AG Eindhoven (NL)
(72) Inventor: VAN EE, Raymond, Eindhoven (NL); AZIZ, Gona Nori, Eindhoven (NL)
(74) Representative: Philips Intellectual Property & Standards

(57) **Abstract**

Disclosed herein is a medical system (100) for providing station specific instructions (150) for a set of stations (102). The execution of the machine executable instructions (140) causes a computational system (106) to control a subject locator (136) for determining (200) the current station and to perform a verification procedure comprising: receiving (202) station specific instructions comprising at least one manual action in response to inputting a received chosen protocol (144), the indication of the current station and an instruction mode (146) into an instruction engine (148); presenting (204) the station specific instructions; controlling (206) a subject measurement system (132); assessing (208) a measure of completion (152); and modifying (212) the instruction mode if (210) the measure of completion meets a predetermined modification criterion (154). The computational system to repeatedly performs the verification procedure until the measure of completion meets (214) a predetermined completion criterion (156).

## Description

### FIELD OF THE INVENTION

The invention relates to medical instruction systems.

### BACKGROUND OF THE INVENTION

When a subject undergoes a medical or imaging procedure, the subject may need to interact with a number of medical devices which should be configured. To perform this, the subject may need to undertake a variety of tasks in preparation. This can be difficult to coordinate and may or may not be successful based upon subject's ability, communication type, and level of personal mobility.

### SUMMARY OF THE INVENTION

The invention provides for a medical system, a method, and a computer program in the independent claims. Embodiments are given in the dependent claims.

In one aspect, a medical system is disclosed. The medical system is configured for providing station-specific instructions for a set of stations. The medical system comprises a memory storing machine-executable instructions and a computational system. Execution of the machine-executable instructions causes the computational system to repeatedly control a subject locator for determining a subject presence at a current station of the set of stations, providing an indication of the current station, and to perform a verification procedure. The verification procedure comprises receiving station-specific instructions for the determined current station in response to inputting a received current chosen protocol, the indication of the current station and an instruction mode into an instruction engine. The station-specific instructions comprise instructions for the subject to perform at least one manual action at the current station. The verification procedure further comprises presenting the station-specific instructions to the subject using an instruction presentation system. The verification procedure further comprises controlling a subject measurement system to measure subject data in response to presenting the station-specific instructions. The verification procedure further comprises assessing a measure of completion of the at least one manual action using the subject sensor data. The verification procedure further comprises modifying the instruction mode if the measure of completion meets a predetermined modification criterion.

Execution of the machine-executable instructions further causes the computational system to repeatedly perform the verification procedure. In case the measure of completion meets a predetermined completion criterion, the station-specific instructions comprise an indication of a station subsequent to the current station in accordance with a sequence of stations. The chosen protocol specifies the sequence of stations.

In another aspect a method of operating a medical system is disclosed. The method comprises repeatedly controlling a subject locator for determining a subject presence at a current station of the set of stations, providing an indication of the current station, and to perform a verification procedure. The verification procedure comprises receiving station-specific instructions for the determined current station in response to inputting a received chosen protocol, the indication of the current station, and an instruction mode into an instruction engine. The station-specific instructions comprise instructions for the subject to perform at least one manual action at the current station. The verification procedure further comprises presenting the station-specific instructions to the subject using an instruction presentation system. The verification procedure further comprises controlling a subject measurement system to measure subject data in response to presenting the station-specific instructions. The verification procedure further comprises assessing a measure of completion of the at least one manual action using the subject sensor data. The verification procedure further comprises modifying the instruction mode if the measure of completion meets a predetermined modification criterion.

The method further comprises repeatedly performing the verification procedure. In case the measure of completion meets a predetermined completion criterion, the station-specific instructions comprise an indication of a station subsequent to the current station in accordance with a sequence of stations. The chosen protocol specifies the sequence of stations.

In another aspect, a computer program comprising machine-executable instructions configured for controlling a computational system to perform the method is disclosed herein.

### BRIEF DESCRIPTION OF THE DRAWINGS

In the following preferred embodiments of the invention will be described, by way of example only, and with reference to the drawings in which:
- Fig. 1: illustrates an example of a medical system.
- Fig. 2: shows a flow chart which illustrates a method of using the medical system of Fig. 1.
- Fig. 3: illustrates an example of a changing room station.
- Fig. 4: illustrates an example of a magnetic resonance imaging station.
- Fig. 5: illustrates an example of a subject path or journey through a healthcare facility.
- Fig. 6: shows a flow chart which illustrates one way of using the instruction engine.

### DETAILED DESCRIPTION OF EMBODIMENTS

Like numbered elements in these figures are either equivalent elements or perform the same function. Elements which have been discussed previously will not necessarily be discussed in later figures if the function is equivalent.

In one example a medical system is configured for providing station-specific instructions for a set of stations. The various stations of the set of stations may take different forms in different examples. In one example, a station may encompass the use of a computer or a handheld computing device at a predetermined time. In this case, the station is independent of a particular location. The vicinity of the subject to a computing or handheld computing device at a particular time may suffice to indicate the location of the subject. In other examples, the various stations of the set of stations may represent physical locations in a medical facility such as a community center, doctor's office, or hospital. The station-specific instructions may provide various types of information to a subject. In some examples the station-specific instructions may provide physical activities or tasks for a subject to perform. This may for example prepare a subject for a particular medical procedure or imaging procedure. In other cases, the station-specific instructions may also include various educational information and ideas which are transmitted to the subject. In some cases, the station-specific instructions may combine a variety of tasks and information which are provided to the subject.

The medical system comprises a memory storing machine-executable instructions and a computational system. The execution of the machine-executable instructions causes the computational system to repeatedly control a subject locator for determining a subject presence at a current station of the set of stations. The subject locator may take different forms in different examples. In one case the subject locator may be a device which registers the physical presence of a subject at a particular location. For example, the subject locator may provide GPS data or Wi-Fi data from a device carried by the subject. In other examples the subject locator may include such things as being logged into a computing device or handheld computing device. For example, if a station is to present information or tasks via a smartphone or other handheld device, logging into this device may determine the subject is present at a current station.

The subject locator may also be used for providing an indication of the current station. For example, the set of stations may be stations which are performed in a particular sequence. The current position of the subject within the set of stations may be determined using the subject locator. In some examples the subject locator is also used to perform a verification procedure on the subject. This verification procedure may comprise receiving station-specific instructions for the determined current station in response to inputting a received chosen protocol, the indication of the current station, and an instruction mode into an instruction engine. The chosen protocol may comprise for example be a determined path through a healthcare facility for the subject. The indication of the current station may specify where in the set of stations the subject is and the instruction mode may be used to determine the type or form of instructions or data which is presented to the subject. The instruction engine may for example be a database or other system for providing data and/or instructions. The instruction engine is able to provide different instructions based on the received chosen protocol, the indication of the current station and the instruction mode. This means for a particular protocol and current station there are multiple station-specific instructions which can be selected for a subject. This may for example be beneficial when the subject has difficulty following or performing the station-specific instructions. For example, they may be presented in a form or language which is able to be interpreted by the subject. This may also introduce the ability to adjust the instruction mode so that the particular type or format of the station-specific instructions are tailored or modified to fit the preferences and abilities of the subject. The station-specific instructions comprise instructions for the subject to perform at least one manual action at the current station. For example, the station-specific instructions could provide detailed tasks or instructions on how to perform this manual action.

The verification procedure further comprises presenting the station-specific instructions to the subject using an instruction presentation system. The instruction presentation system may take different forms in different examples. In one case it may be a screen or display on a computer or handheld computing device. In other examples it may provide information via a projector. In some cases, the station-specific instructions may change the lighting or ambience of a particular station. In further examples, the station-specific instructions may be presented using a multimedia presentation. The configuration of the station-specific instructions may change with the instruction mode. For example, the way that the instructions are presented as well as the ambience may be modified or changed using the instruction mode. The verification procedure further comprises controlling a subject measurement system to measure subject data in response to presenting the station-specific instructions. The subject data may take different forms in different examples also. In some cases, it may be data which is able to measure a physical property of a subject or a physiological state. In some cases, the subject data may also comprise data from a questionnaire or other information typed into a user interface.

The verification procedure further comprises accessing a measure of completion of the at least one manual action using the sensor data. The station-specific instructions are presented to the subject and contain instructions for completing at least one manual action. The subject data is used to calculate a measure to determine if the at least one manual action was completed or how well it was performed. Performing the verification procedure further comprises modifying the instruction mode if the measure of completion meets a predetermined modification criterion. For example, if the subject data indicates that the subject was unable to perform the at least one manual task in response to receiving the station-specific instructions then the instruction mode may be changed and the type or modality of instructions is changed to one that may better fit or be more understandable by the subject.

Execution of the machine-executable instructions further causes the computational system to repeatedly perform the verification procedure. In case the measure of completion meets a predetermined completion criterion the station-specific instructions comprise an indication of a station subsequent to the current station in accordance with the sequence of stations. In this feature when the predetermined completion criterion is met this may indicate that the subject was able to successfully perform the at least one manual action at the current station. The system may then for example provide details on how to proceed to the subsequent station. The chosen protocol specifies the sequence of stations.

There is an unmet need associated with conducting a successful medical exam. This is to provide a patient with adaptive personalized instructions during the patient's journey from home, through the hospital, to the medical exam room in a seamlessly integrated way. Such adaptive personalized instructions are particularly appropriate when a patient is anxious for the upcoming medical exam. A potential reason for this unmet need that expectation management, efficiency, and the patient's mood can be improved along the way which, in turn, decreases costs (enhanced exam results with less repetition) and period of time associated with the medical exam. Examples may provide a means of meeting this unmet need by providing an instruction engine with a changeable communication mode that can be adapted to a subject.

Consider for example an MR-scan: Patients, and particularly children, are often anxious (possibly caused by fear that may be related to claustrophobia) for the small bore of an MR-scanner. Patients and, as noted above, particularly staff would like to have the possibility to communicate adaptive personalized information during the patient's journey from home, through the hospital, to an exam room.

In addition, hospital management requires that such adaptive personalized instructions can be provided in a seamless way via systems that are integrated and connected across hospital rooms (e.g. for waiting, dressing, sedation, etc.) and devices for those procedures where multiple devices are being used. In other words, integrated solutions, as opposed to point solutions, are being requested.

Adaptive personalized instructions could be given by either a nurse or a hospital assistant but those are not available in the current workflow.

Station specific instructions could be given by an avatar in the form of a cartoon character that is presented via a projection screen and/or an app on a smart phone. Station specific instructions can also be given or embedded in an ambient environment.

Examples may provide an instruction engine that provides adaptive personalized information during a patient's journey from home, through the hospital, to an exam room in the form of station specific instructions. In addition, these adaptive personalized instructions can be provided in a seamless way via systems that are integrated and connected (for example via Wi-Fi) across hospital rooms and devices.

Thus, adaptive personalized instructions (the station specific instructions) may improve workflow and quality of a medical exam because it may assist in saving time and costs.

In another example, the sequence of stations comprises a medical machine station with a medical machine. A medical machine as used herein encompasses a machine which may be used for a medical purpose. This may include such things as medical imaging systems as well as machines for performing a task or use on a subject. Execution of the machine-executable instructions further causes the computational system to modify configuration of the medical machine using the subject data acquired at previous stations. The subject data may for example indicate how well the subject was able to perform the at least one manual action as well as any supplementary educational materials which may have been presented to the subject. The subject data may then be used to measure the effect of going through the set of stations on a subject. Using this information the configuration of the medical machine is modified.

In another example, the medical machine comprises a medical imaging system. A medical imaging system as used herein may encompass such things as a tomographic medical imaging system such as computed tomography or magnetic resonance imaging system. It may also include other medical imaging modalities such as ultrasound or a digital X-ray or fluoroscope. In this example, the configuration of the medical imaging system is modified using the subject data. This may have several examples, in one case the operation of the medical imaging system may be tailored or modified to fit the subject. In some examples the collection of the subject data may free a human operator from the need to collect data and may provide for faster medical imaging with possibly lower costs due to a lower human interaction.

In another example the set of stations comprises a mockup of the medical imaging system. The mockup of the medical imaging system may for example be a full sized medical imaging system and may enable the subject to become accustomed to operating or performing tasks or getting into the medical imaging system. In other examples, the mockup of the medical imaging system may be small or of a reduced size such as would be a toy for a child. This may for example enable the subject to become better acquainted with or more comfortable around the medical imaging system. The at least one physical action may be for example to perform various tasks or operations using the mockup of the medical imaging system. This may for example make subjects, and in particular children, more comfortable around a real medical imaging system and may result in higher image quality.

The station-specific instructions of the mockup of the medical imaging system comprise training instructions for the medical imaging system. Execution of the machine-executable instructions are configured such that the subject data acquired during performance of the training instructions is used to modify the configuration of the medical imaging system.

In another example, execution of the machine-executable instructions further causes the computational system to acquire medical image data by controlling the medical imaging system with the modified medical imaging system control commands. This example may be beneficial because the medical imaging system was configured using the subject data. This may for example lead to higher quality medical images.

In another example, the medical system is a magnetic resonance imaging system. The modified medical imaging system control commands are pulse sequence commands. The modifications to the pulse sequence commands comprise any one of the following: a selection of a compressed sensing compression ratio, a selection of a motion correction protocol, a modification of the pulse sequence type, commands to cause breathing exercises in the bore of the magnetic resonance imaging system, and a number of radio frequency pulses used in the pulse sequence, and combinations thereof. This example may be beneficial because it may provide for a means of modifying the magnetic resonance imaging system such that it performs better imaging with a particular subject.

In another example, the sequence of stations comprises any one of the following locations, a waiting room station, a dressing room station, a sedation station, an imaging room station, a scanner bore, a wake up room station, and combinations or multiple thereof. This example may be beneficial because the at least one manual action for each of these various locations may be presented to a subject in a tailored and effective way. This may for example enable the subject to perform better during an imaging procedure or to proceed through the various stations within a hospital or medical facility more rapidly.

In another example, the memory further comprises a convolutional neural network configured to output of clothing classification in response to receiving a subject image. This may for example encompass receiving a subject image and then classifying the type of clothes that the subject is wearing or depicted in the subject image. The subject measurement system comprises a camera. Execution of the machine-executable instructions further causes the computational system to acquire the subject image by controlling the camera. Execution of the machine-executable instructions further causes the computational system to determine the clothing classification by inputting the subject image into the convolutional neural network. The subject data comprises the clothing classification. This may for example be useful in the situation where a subject needs to disrobe or change clothes before a medical procedure or before using a medical imaging system such as a magnetic resonance imaging system or computed tomography system. This example may enable the automated determination if the subject has successfully changed from street clothing into for example, a dressing gown.

The convolutional neural network may be one of the standard convolutional neural networks that is used for image recognition. For example, the AlexNet, VGGNet, GoogLeNet/Inception, ResNet, and DenseNet architectures may be used. The convolutional neural network may be trained by supplying images which are labeled as to the type of clothing that a subject or person in the image is wearing. The convolutional neural network can then be trained using a deep learning process.

In another example the instruction presentation system comprises any one of the following: a light amount control at the current station, a voice generation system at the current station, a room control at the current station, a video display system, an augmented reality device, a virtual reality device, and combinations thereof. In various examples the instruction presentation system may provide various types of audio, visual and even possibly tactile feedback to a subject.

In another example, the instruction presentation system comprises a handheld computing device and/or wherein the subject location locator comprises the handheld computing device. For example, a subject may be able to register their cell phone or smartphone with the medical system. This may provide localized data as to the location or position of the subject relative to various stations.

In another embodiment the sequence of stations comprises a preparation room or preparation app. The preparation room or preparation app may be used to provide information and instructions for the subject at the beginning of the sequence of stations or near the beginning. A preparation room may for example be a room in a hospital, a medical center, a community center, a place of worship or other location where people could gather and receive information or instructions collectively. In the case where the subject is not able to visit a preparation room a specialized piece of software, like a preparation app, may be installed on the subject's personal computing device such as a smartphone to enable the same type of instruction.

In another example, the set of stations comprises an aftercare room or app. The aftercare room may for example be a room at a hospital, a medical facility, a community center, or a place of worship, where information may be provided to a group of subjects after a medical procedure or medical imaging process has taken place. Alternatively, when an aftercare room is not available, an aftercare app may be installed onto the subject's personal computing device such as smartphone, which may enable similar information to be presented as if they were in an aftercare room.

In another example, execution of the machine-executable instructions is configured such that the subject data collected at the preparation room or preparation app comprises a selection of a presentation theme for use of a subsequent station in the sequence of stations. The selection of a presentation theme may for example better enable the subject to follow the patient-specific instructions.

In another example, execution of the machine-executable instructions is configured such that the subject data collected at the preparation room or preparation app comprises physical mobility data collected in response to a physical ability questionnaire and/or in response to the presentation of the at least one manual action, the at least one manual action comprising physical mobility test instructions. This may be very beneficial because it may provide a means of automatically assessing the physical mobility of a subject before proceeding to further stations. This may enable the tailoring of the manual tasks to fit the physical mobility of the subject and may enable the subject to better traverse the set of stations.

In another example, execution of the machine-executable instructions is configured such that the subject data collected at the preparation room or preparation app comprises breathing data collected during the presentation of breath hold training instructions. For example, the at least one manual action may comprise instruction on how to perform a breath hold during a medical imaging procedure. The subject data may comprise breathing data and this information on the subject's ability to perform a breath hold may be used for example to modify the parameters using during a medical imaging procedure. For example, if a subject is better able to perform breathing hold then a magnetic resonance imaging protocol can be chosen to match how well the subject can hold their breath.

In another example the subject data comprises subject sensor data. The subject sensor data is data which is descriptive of a physiological state of the subject or is related to a mechanical or physical property of the subject.

In another example the subject measurement system comprises any one of the following for acquiring the subject sensor data: a wearable sensor, a station-specific camera, a station-specific microphone, a physiological sensor, a heart rate monitor, an accelerometer, a step counter, a respiratory sensor, and combinations thereof. These examples may be particularly beneficial because they may be able to infer a large degree of accuracy as to the subject's physical state as well as the subject's completion of the at least one manual action. For example, an accelerometer and/or step counter may be useful in determining if the subject is actually performing the at least one manual action.

In another example, the medical system further comprises an operator interface configured for enabling speech communication with the subject and/or for overriding the medical system. Overriding the medical system may comprise any one of the following: halting the medical system, restarting the medical system, causing the medical system to repeat one or more of the set of stations for the subject, manually entering or editing the subject data, manually setting the current station, skipping the current station, skipping subsequent stations, and combinations thereof. This example may be useful because it may provide for a means of correcting or modifying the medical system to fit the particular needs of a subject or when the medical system has an error or is not functioning properly.

In another example, the instruction engine is a relational database or a lookup table. This may be beneficial because it may provide for a very efficient means of providing the different station-specific instructions based on the particular chosen protocol, the indication of a current station and the particular instruction mode. The lookup table or relational database can hold commands or data which is appropriate for the various stations and the various modes of training or interacting with the subject.

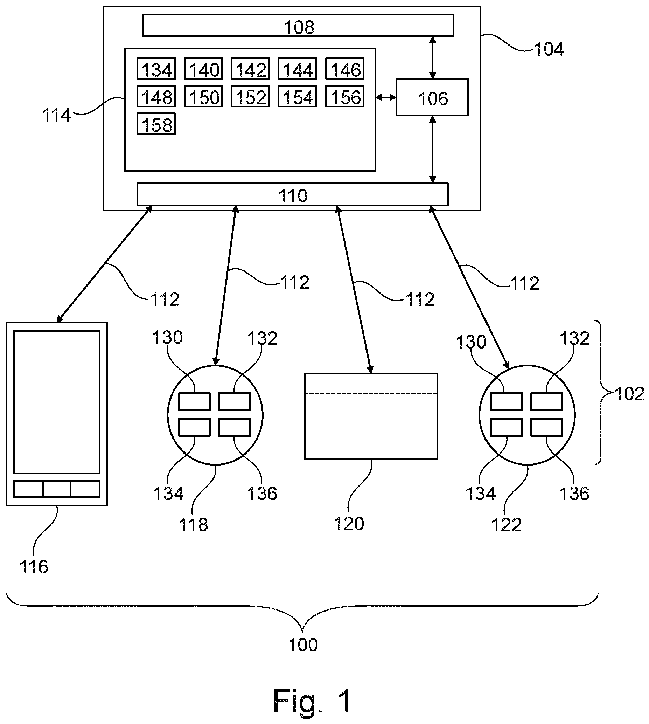

Fig. 1 illustrates an example of a medical system 100. The medical system 100 comprises a set of stations 102 and a server 104. The server 104 comprises a computational system 106 that is connected to a user interface 108, a network interface 110 and a memory 114. The network interface 110 is able to form a network connection 112 with the various members of the set of stations 102.

The set of stations 102 may be formed from different devices and/or locations. The set of stations 102 is shown as comprising a handheld device 116 such as a smartphone, a location at a healthcare facility 118, a medical imaging system 120, and a location at a healthcare facility 122. The stations in the set of stations 102 are therefore comprised of not only physical locations 118, 120, 122 but also opportunities to use a computing device such as the handheld device 116.

The locations at the healthcare facility 118, 122 are shown as comprising an instruction presentation system 130, a subject measurement system 132, storing subject data 134 acquired with the subject measurement system 132, and a subject locator 136. The subject locator 136 may for example be a system which detects the presence of a subject. In the case of the handheld device 116 the subject locator 136 could for example be an indication that the subject has logged into and is currently using the handheld device 116.

The memory 114 of the server 104 is shown as storing subject data 134 that was obtained from one of the set of stations 102 as well as machine-executable instructions 140. The machine-executable instructions 140 enable the computational system 106 to perform various computational and data processing tasks. The memory 114 is further shown as containing an indication of the current station 142 that was determined using one of the subject locator 136. The memory 114 is further shown as containing a chosen protocol 144. The chosen protocol 144 may for example be a pre-selected protocol assigned to a subject who is going to be entering a healthcare facility. The memory 114 is further shown as containing an instruction mode 146 that was selected for the subject. The instruction mode 146 could for example be a default instruction mode 146 or associated with medical records of the subject. The memory 114 is further shown as containing an instruction engine 148 that is able to provide station-specific instructions 150 in response to inputting the chosen protocol 144, the instruction mode 146 and the indication of the current station 142. The instruction engine 148 may for example be a relational database or a lookup table.

The memory 114 is further shown as containing station-specific instructions 150 that contain instructions on how to perform at least one manual action at the current station for the subject. The station-specific instructions 150 were received from the instruction engine 148 and would then be pushed out along the network connection 112 to the current instruction station. The subject data 134 could be compared to a predetermined modification criterion 154 to determine if the instruction mode 146 is correct. If it is not, the instruction mode 146 can be changed or shifted and this can be used to then obtain new station-specific instructions 150 that may be more understandable or easier to follow for the subject.

The memory 114 is further shown as containing a predetermined completion criterion which may be compared to the measure of completion 152 to determine if the subject is finished with the current station. If the subject is determined to be finished then the indication of a station subsequent to the current station 158 may be provided. These for example may be instructions for the subject on how to travel to the next station or when to login to the handheld device 116.

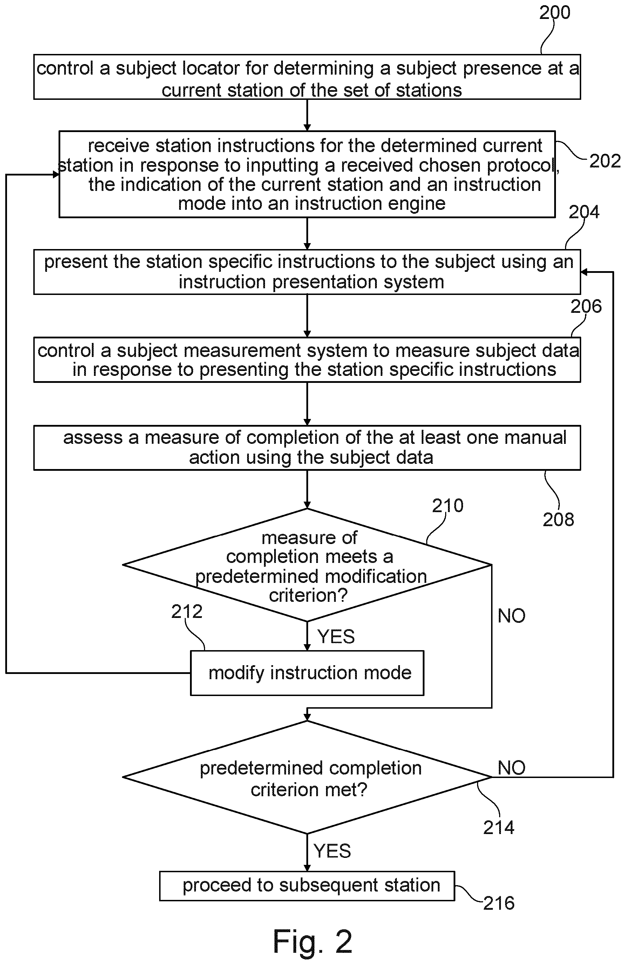

Fig. 2 shows a flowchart which illustrates a method of operating the medical system 100. In step 200, the subject locator 136 is controlled to determine the subject presence at a current station of the set of stations 102. In step 202, the station-specific instructions 150 are received in response to inputting the determined current station, the received chosen protocol 144 and an instruction mode 146 into the instruction engine 148. These may then for example be pushed across the network connection. In step 204, the station-specific instructions 150 are presented to the subject using the instruction presentation system 130. In step 206, the subject measurement system 132 is controlled to measure the subject data 134. In step 208, the measure of completion 152 is assessed for the at least one manual action using the subject data 134. Step 210, is a decision box and the question is does the measure of completion meet a predetermined modification criterion. If the answer is yes then the method proceeds to block 212 where the instruction mode is modified. After this the method returns to step 202 to retrieve the station-specific instructions 150 from the instruction engine 148 after the instruction mode 146 has been updated. Returning back to block 210, if the answer is no, then the method proceeds to block 214. In block 214 the question is has the predetermined completion criterion been met. If the answer is no the method proceeds back to block 204. If the answer is yes the method proceeds to block 216 where the subject is instructed to proceed to the subsequent station. The method illustrated in Fig. 2 is for a single station of the set of stations 102.

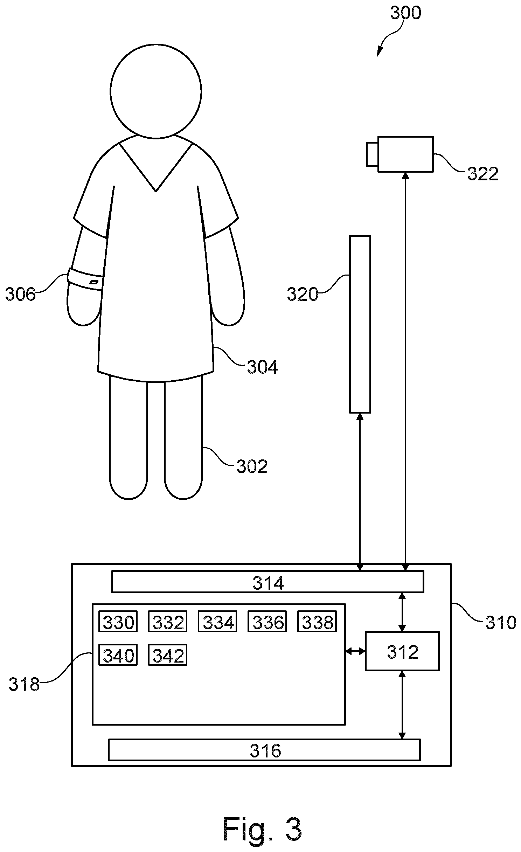

Fig. 3 illustrates an example of a changing room station 300. A subject 302 wearing a dressing gown 304 is shown. The subject 302 is also wearing a bracelet 306. If the subject 302 is about to have a magnetic resonance imaging examination then the bracelet 306 could be a hazard.

The changing room station 300 comprises a local computer 310 with a local computational system 312 that is in communication with a local hardware interface 314, a local network interface 316, and a local memory 318. The local hardware interface 314 is in communication with a presentation system 320 and a camera system 322. The presentation system 320 is able to provide instructions and/or videos for the subject 304. In this case the station-specific instructions 340 may instruct the subject 302 to change into the dressing gown 304 before the magnetic resonance imaging examination.

The local memory 318 has local machine-executable instructions 330 that enable the local computational system 312 to perform various analysis and data processing tasks. The local memory 318 is further shown as containing a convolutional neural network 332 that is able to take a subject image 334 recorded by the camera system 322 of the subject 304. It is able to classify different objects within the subject image 334. The memory 318 is shown as containing a subject image 334 recorded with the camera system 322 of the subject 304. The subject image 334 is input into the convolutional neural network 332 and it receives a classification of a dressing gown 336 and a classification of a bracelet 338. The memory 318 is further shown as containing station-specific instructions 340 that instructed the subject 304 to change into the dressing gown 304 and to remove all jewelry. The classification of the bracelet 338 indicates that the subject 302 did not follow the station-specific instructions 304 properly. The memory 318 then contains instructions to remove the bracelet 342 which may be presented using the presentation system 320. The changing room station 300 is able to automatically identify if the subject 302 is in the dressing gown 304 as well as in some cases identify if the subject 302 did not follow these instructions properly.

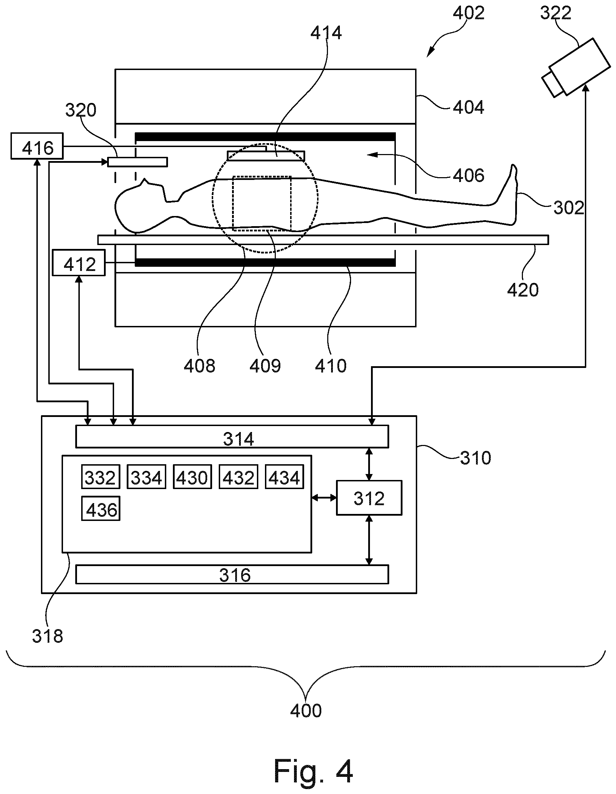

Fig. 4 illustrates an example of a magnetic resonance imaging station 400. This is one of the sets of stations that comprises a magnetic resonance imaging system 402. In this context the local computer system 310 is configured for controlling the magnetic resonance imaging system 402.

The magnetic resonance imaging system 402 comprises a magnet 404. The magnet 404 is a superconducting cylindrical type magnet with a bore 406 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 406 of the cylindrical magnet 404 there is an imaging zone 408 where the magnetic field is strong and uniform enough to perform magnetic resonance imaging. A field of view 409 is shown within the imaging zone 408. The measured k-space data is acquired for the field of view 409. A subject 302 is shown as being supported by a subject support 420 such that at least a portion of the subject 302 is within the imaging zone 408 and the field of view 409. The measured k-space data that will be acquired is therefore for performing a head or brain scan.

Within the bore 406 of the magnet there is also a set of magnetic field gradient coils 410 which is used for the acquisition of measured k-space data to spatially encode magnetic spins within the imaging zone 408 of the magnet 404. The magnetic field gradient coils 410 are connected to a magnetic field gradient coil power supply 412. The magnetic field gradient coils 410 are intended to be representative. Typically, magnetic field gradient coils 410 contain three separate sets of coils for spatial encoding in three orthogonal spatial directions. A magnetic field gradient power supply supplies current to the magnetic field gradient coils. The current supplied to the magnetic field gradient coils 410 is controlled as a function of time and may be ramped or pulsed.

Adjacent to the imaging zone 408 is a radio frequency coil 414 for manipulating the orientations of magnetic spins within the imaging zone 408 and for receiving radio transmissions from spins also within the imaging zone 408. The radio frequency antenna may contain multiple coil elements. The radio frequency antenna may also be referred to as a channel or antenna. The radio frequency coil 414 is connected to a radio frequency transceiver 416. The radio frequency coil 414 and radio frequency transceiver 416 may be replaced by separate transmit and receive coils and a separate transmitter and receiver. It is understood that the radio frequency coil 414 and the radio frequency transceiver 416 are representative. The radio frequency coil 414 is intended to also represent a dedicated transmit antenna and a dedicated receive antenna. Likewise, the transceiver 416 may also represent a separate transmitter and receiver. The radio frequency coil 414 may also have multiple receive/transmit elements and the radio frequency transceiver 416 may have multiple receive/transmit channels. The transceiver 416 and the gradient controller 412 are shown as being connected to the hardware interface 3146 of the computer system 310.

In this example there is again a camera system 322 that contains a subject image 334 which may be input into the convolutional neural network 332. In this case the convolutional neural network 332 could for example be used to identify hazardous materials or if the subject 302 is positioned properly in the magnetic resonance imaging system 402. In some examples the presentation system 320 could be used to provide breathing instructions or breath hold instructions to the subject 302 during the magnetic resonance imaging procedure.

The local memory 318 is shown as containing subject data from previous stations 434. This subject data from previous stations 434 contains data descriptive of the subject 302 and the subject's preparation for the magnetic resonance imaging procedure. It may take pulse sequence commands 432 and generate modified pulse sequence commands 436. The modifications to the pulse sequence commands could comprise any one of the following: a selection of a compressed sensing compression ratio, a selection of a motion correction protocol, a modification of the pulse sequence type, commands to cause breathing exercises in a bore of the magnetic resonance imaging system, a number of radiofrequency pulses used in the pulse sequence, and combinations thereof.

The memory 318 is further shown as containing k-space data 438 that was acquired by controlling the magnetic resonance imaging system 402 with the modified pulse sequence commands 436. The memory 318 is further shown as containing a magnetic resonance image 440 that was reconstructed from the k-space data 438.

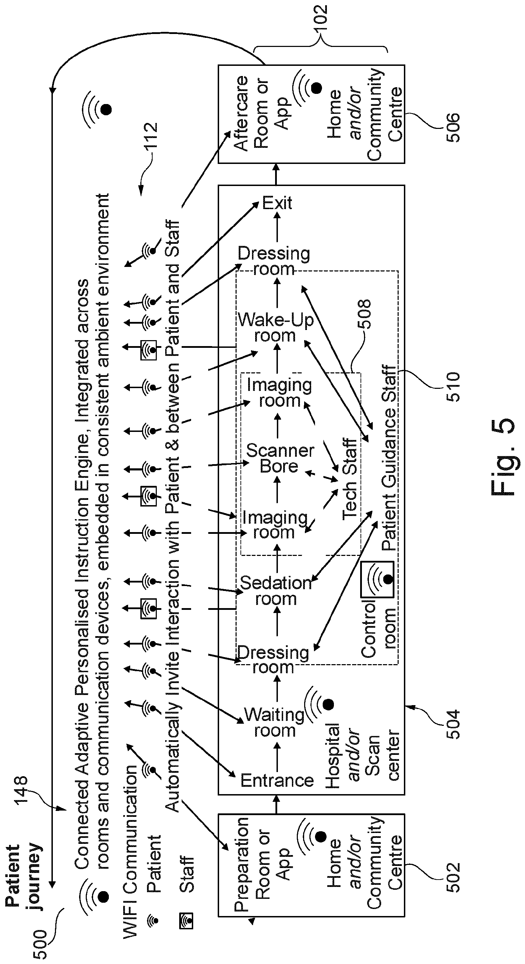

Fig. 5 illustrates a subject path or journey 500 through a healthcare facility. This path represents one means of using the medical system 100 illustrated in Fig. 1. There is a preparation room or app 502, various members of the set of stations 504 and also an aftercare room or app 506. 502, 504, and 506 represent one example of the set of stations 102. Via a network connection 112 the set of stations 102 are in communication with the server 104 which has an instance of the instruction engine 148. As the subject follows the path 500 it goes into various stations 102. At the appropriate station the station-specific instructions 150 are provided by the instruction engine 148. This system is additionally shown as having a mechanism for technical staff 508 to communicate directly with the subject as well as patient guidance staff 510 also having a means of communicating with the subject 302 also. This communication means 508, 510 may also be used for modifying the behavior and controlling of the medical system 100.

The patient journey 500 usually starts at home using an app 502, but it can also start in a community center possibly using a preparation room. Subsequently the patient arrives at the entrance of the hospital (or a general specialized exam center). The journey then sequential goes through 504: the entrance, waiting room, dressing room, sedation room (optional), Imaging room, and Scanner Bore of a medical imaging system. Then after the scan the journey goes from: the scanner, wake-up room (optional), dressing room, control room, exit of hospital and/or scan center, Aftercare 506 which can be at home or at a community center. For a follow up visit the journey starts again at the left side.

During the journey the patient is provided with appropriate information in a consistent ambient environment. The information, as well as the ambient environment, can be personalized and adaptive to make the patient ready for the next step in the procedure. The information can be automatically selected based upon measured signals about the patient and/or based upon look up tables where information is retrieved for the patient.

In order to be adaptive, the patient may be automatically invited to interact with the instruction engine.

The control room deserves extra attention. Here both technical staff and other patient guidance staff members are able to have direct communication with the patient through real-time speech. Based upon this staff-patient communication the staff members can adjust or overrule instructions of the instruction engine, as well as the ambient environment provided by the engine. The patient journey can include repeated visits to the hospital.

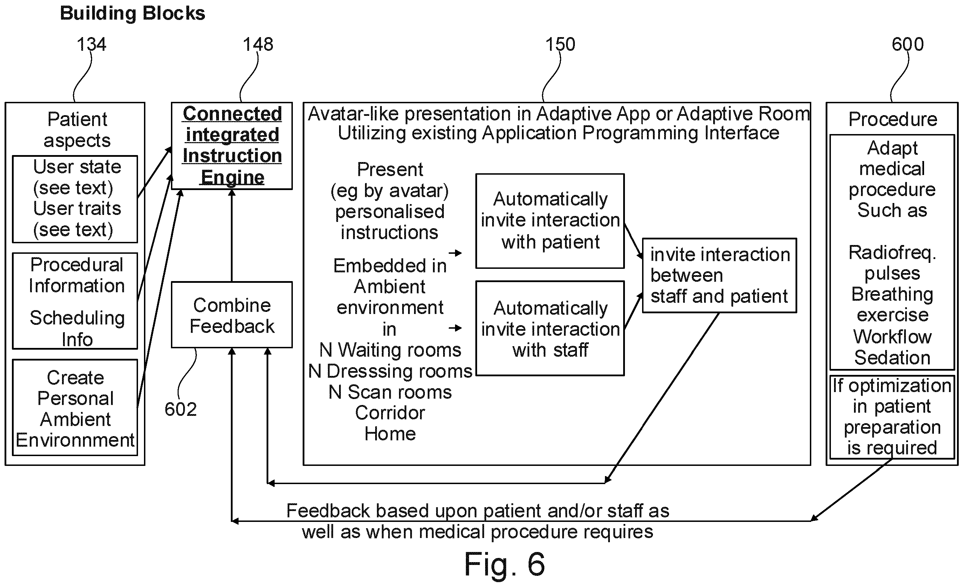

Fig. 6 shows an additional flowchart which illustrates one way of using the instruction engine 148. Subject data 134 which may comprise various aspects is part of the data which is fed into the instruction engine 148. The subject data could additionally include data such as the: user state (e.g. physiology, self-report), scheduling info; user traits (e.g. personality), type of scan procedure, and treatment plan. The instruction engine 148 may combines input from the patient on user state and user trait, as well as procedural information (e.g. for knee or brain scan). It also uses as input the user's preferred ambient environment. The output of the instruction engine may be presented to the patient via either an avatar or an app, in a way that is consistent across rooms and devices. In addition, the patient may be invited to interact with the app, and to interact with staff if required. The result from the interactions is fed back to the instruction engine in order to optimize its instructions. Based upon all collected information and patient state, the medical procedure can be adapted and optimized. If necessary, in case optimization in patient preparation is required, this information is fed back to the instruction engine.

The instruction engine 148 may provide station-specific instructions 150 which may result in subject data 134 being measured. This subject data may be used for providing procedural data 600 which may be used for such things as adapting the behavior of a medical procedure or adapting the control commands for a medical apparatus or a medical imaging system. Feedback from one of the station-specific instructions 150 are performed and when the procedural data 600 is executed on the medical machine may result in feedback 602 being fed back into the instruction engine 148.

In examples, beginning at home the invention we here disclose provides instructions/assistance to a patient on what can be expected for successive steps regarding the upcoming medical exam/procedure. This information will be provided within a pre-selected ambient environment. A seamless story line is being generated from home to the moment of entering the hospital all the way into the treatment/diagnostic room, seamlessly making the transition from preparation phase to actual exam phase. The user is presented with the instructions from the engine by utilizing an interface (projected avatar, phone, or a presented ambient environment) seamlessly during the hospital journey, in the waiting room, in the dressing room, in the exam room, and inside the scanner. In addition, after the scan aftercare can be provided at home.

In some examples, the station specific instructions can be presented in the form of a story based upon preselection by either a medical specialist or the patient. Alternatively, the story to be unfolded can be adapted based upon measured signals about the patient's state as well as on aspects about the workflow. Each room that the patients visits (waiting, dressing, exam, recover room) may possibly the same consistent ambient environment part of the unfolding story.

In some examples mixed reality goggles and/or virtual reality devices can be used to optimally present immersive ambient environments in which the information from the instruction engine is being presented.

In some examples, the subject is further guided by the preselected avatar during the journey through the hospital. This time the content presented is adaptive and personalized based on the actual user state (e.g. physiology, Natural Language Processing, and/or self-report). The instruction engine will automatically instruct and prepare the patient for the upcoming examination (e.g. from a normative data base).

In some examples an avatar on the app accompanies the patient through the journey all the way to the waiting room and can act as a personal coach to the patient by automatically responding to questions (eg from a normative data base) about the upcoming exam, by making the story incre4asingly engaging, and if requested by adapting the ambient environment.

In some examples, the subject may be guided to the dressing room. Interaction between staff and patient is invited. The normal procedure (such as no metal on the body) can be explained once again to the patient and the staff can check whether instructions have been successfully followed. Such interaction can be particularly appropriate when the recorded psychological-mood-state signals about the patient indicate that e.g. anxious behavior may become apparent. The instruction engine can retrieve optimal information for such behavior from a normative data base. Alternatively, a staff member can overrule the automated information by selecting specific information that is regarded as being optimal for the patient at a specific moment.

In some examples, the instruction engine may note notes exceptional behavior of the patient, the engine will invite medical staff to have direct communication with the patient via speech or via a real nurse.

In some examples, there may be guidance through the beginning of a sedation procedure in a specifically devoted room with the consistent ambient environment present. Sedation will only take place if this is considered to be necessary for a successful scan. If the patient is completely sedated medical guidance staff will bring the patient to the scanner room.

In some examples, there is no sedation the instruction engine will continue to guide the patient from the dressing room to the scanner room. The instruction engine can automatically adapt content to reach the desired patient state for the exam. In addition, a guiding nurse can interact with the instruction engine to make last minute changes to the ambient environment.

In some examples, a similar procedure can be applied when the patient is in the bore of the scanner. Again, the instruction engine can automatically adapt content to keep/reach the desired patient state for the exam. Again, technical staff can interact with the instruction engine to adapt to the ambient environment. For some specific cardiac exams, a certain breathing state is preferred for which specific preselected (existing in the prior art) sensory information can be presented (such a visual breathing patterns).

In some examples, the scanning procedure (including the waking up from sedation in case of sedation), either on the way to the dressing room, or in the dressing room, the instruction engine can give automated information, and/or ambient content as well as a further unfolding story, to make the patient relaxed and to tell the patient about next steps. The guidance staff has again the possibility to overrule the automated instructions.

In some examples, the dressing room to the exit of the hospital, automatic instructions can be provided by the instruction engine about subsequent appointments. This is the last moment at the hospital site that the patient can interact with the instruction engine by Wi-Fi.

In some examples, at the end the subject can use the app (connected by Wi-Fi) either at home or at a community center in order to make use of aftercare. The patient is once again guided by the preselected avatar via an app. Key aspect is again that the content presented through the projected avatar is adaptive and personalized. If desired, the patient can re-select both another (or new) avatar, and another (or new) ambient environment. In a sub-embodiment the Instruction Engine does automatically provide advice on aftercare and on what can be expected for preparation for a follow-up hospital visit. In another sub-embodiment appointments can be automatically made if necessary. In addition, in another embodiment the patient is invited to pose questions to the instruction engine. In addition, feedback about the procedure can automatically be invited by the instruction engine. The actual user state (e.g. physiology, Natural Language Processing, and/or self-report) could be taken into account in optimizing the information provided.

In some examples, the patient's state is being assessed using existing methods (e.g. wearable technology, vital signs camera, Natural Language Processing, self-report, etc).

In some examples, the trait aspects of a patient are collected using existing methods [see ref 1]. Reference contains 5 traits for describing individual differences in one's personality. The 5 traits in this approach consist of: Agreeableness; conscientiousness; extraversion; neuroticism; openness to experience. Hospitals can use these traits to predict how patients relate to others. Also, it can be used for understanding how patients might handle stress. Each of the 5 personality factors is composed of a range between two extremes.

In some examples, the location of the patient in the hospital (e.g., RF system, geo location, or smart monitoring) is used to update content. Content could also change if the patient is not following the correct route.

In some examples, the treatment plan for the patient is being presented in order to be able to add procedure specific information. For a knee scan the provided information is different than for a heart scan.

In some examples, the content of the ambient environment selected/created by the patient and or staff is retrieved.

In some examples, information on environment, procedure, location, and on the patient's state is combined by the connected and integrated instruction engine in order to provide optimal information to the patient.

In some examples, information can be presented by an avatar that is present on a smart phone via an app. The avatar is also embedded within the ambient environment. This ambient environment is continuously and consistently present along the patient's journey and can be presented in all rooms that will be visited by the patient.

In some examples, interaction of an app with the subject and with the staff is invited.

In some examples, interaction of patient and staff is invited.

In some examples, via a feedback loop, the instruction engine automatically adapts the content of information and of the ambient environment.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: set of stations
- 104: server
- 106: computational system
- 108: user interface
- 110: network interface
- 112: network connection
- 114: memory
- 116: handheld device
- 118: location at healthcare facility
- 120: medical imaging system
- 122: location at healthcare facility
- 130: instruction presentation system
- 132: subject measurement system
- 134: subject data
- 136: subject locator
- 140: machine executable instructions
- 142: indication of the current station
- 144: chosen protocol
- 146: instruction mode
- 148: instruction engine
- 134: subject data
- 150: station specific instructions
- 152: measure of completion
- 154: predetermined modification criterion
- 156: predetermined completion criterion
- 158: indication of a station subsequent to the current station
- 200: control a subject locator for determining a subject presence at a current station of the set of stations, providing an indication of the current station
- 202: receive station specific instructions for the determined current station in response to inputting a received chosen protocol, the indication of the current station and an instruction mode into an instruction engine
- 204: present the station specific instructions to the subject using an instruction presentation system
- 206: control a subject measurement system to measure subject data in response to presenting the station specific instructions
- 208: assess a measure of completion of the at least one manual action using the subject sensor data
- 210: measure of completion meets a predetermined criterion?
- 212: modify the instruction mode
- 214: predetermined completion criterion met?
- 216: proceed to subsequent station
- 300: changing room station
- 302: subject
- 304: dressing gown
- 306: bracelet
- 310: local computer
- 312: local computational system
- 314: hardware interface
- 316: network interface
- 318: memory
- 320: presentation system
- 322: camera system
- 330: local machine executable instructions
- 332: convolutional neural network
- 334: subject image
- 336: classification of dressing gown
- 338: classification of bracelet
- 340: station specific instructions
- 342: instructions to remove bracelet
- 400: magnetic resonance imaging station
- 402: magnetic resonance imaging system
- 404: magnet
- 406: bore of magnet
- 408: imaging zone
- 409: field of view
- 410: magnetic field gradient coils
- 412: magnetic field gradient coil power supply
- 414: radio frequency coil
- 416: transceiver
- 420: subject support
- 430: machine executable commands
- 432: pulse sequence commands
- 434: subject data from previous stations
- 436: modified pulse sequence commands
- 438: k-space data
- 440: magnetic resonance image
- 500: subject path through healthcare facility
- 502: preparation room or app
- 504: various members of set of stations
- 506: aftercare room or app
- 508: technical staff
- 510: patient guidance staff

## Claims

1. A medical system (100) for providing station specific instructions (150) for a set of stations (102), the medical system comprising a memory storing machine executable instructions (140) and a computational system (106), wherein execution of the machine executable instructions causes the computational system to repeatedly control (200) a subject locator (136) for determining (200) a subject presence at a current station of the set of stations, providing an indication of the current station, and to perform a verification procedure comprising:
- receive (202) station specific instructions for the determined current station in response to inputting a received chosen protocol (144), the indication of the current station and an instruction mode (146) into an instruction engine (148), the station specific instructions comprising instructions for the subject to perform at least one manual action at the current station;
- present (204) the station specific instructions to the subject using an instruction presentation system (130, 320);
- control (206) a subject measurement system (132, 322) to measure subject data (134) in response to presenting the station specific instructions;
- assess (208) a measure of completion (152) of the at least one manual action using the subject sensor data;
- modify (212) the instruction mode if (210) the measure of completion meets a predetermined modification criterion (154);
wherein execution of the machine executable instructions causes the computational system to repeatedly perform the verification procedure, wherein in case the measure of completion meets (214) a predetermined completion criterion (156), the station specific instructions comprise an indication of a station subsequent (216) to the current station in accordance with a sequence of stations, wherein the chosen protocol specifies the sequence of stations.

2. The medical system of any one of the preceding claims, wherein the sequence of stations comprises a medical machine station (400) with a medical machine (402), wherein execution of the machine executable instructions further causes the computational system to modify a configuration of the medical machine using the subject data acquired at previous stations.

3. The medical system of claim 2, wherein medical machine comprises a medical imaging system (402).

4. The medical system of claim 3, wherein the set of stations comprises a mockup of the medical imaging system, wherein the station specific instructions of the mockup comprise training instructions for the medical imaging system, and wherein execution of the machine executable instructions are configured such that subject data acquired during performance of the training instructions is used to modify the configuration of the medical imaging system.

5. The medical system of claim 3 or 4, wherein execution of the machine executable instructions causes the computational system to acquire medical image data (432) by controlling the medical imaging system with the modified medical imaging system control commands (436).

6. The medical system of claim 3, 4, or 5, wherein the medical system is a magnetic resonance imaging system (402), wherein the modified medical imaging system control commands are pulse sequence commands (432), and wherein the modifications to the pulse sequence commands comprise any one of the following: a selection of a compressed sensing compression ratio, a selection of a motion correction protocol, a modification of the pulse sequence type, commands to cause breathing exercises in a bore of the magnetic resonance imaging system, a number of radiofrequency pulses used in the pulse sequence, and combinations thereof.

7. The medical system of any one of the preceding claims, wherein the sequence of stations comprises any one of the following locations: a waiting room station, a dressing room station (300), a sedation station, an imaging room station (400), a scanner bore (406), a wake up room station, and combinations thereof and/or multiple thereof.

8. The medical system of any one of the preceding claims, wherein the memory further comprises a convolutional neural network (332) configured to output a clothing classification (336) in response to receiving a subject image (334) as input, wherein the subject measurement system comprises a camera, wherein execution of the machine executable instructions further causes the computational system to:
- acquire the subject image by controlling the camera; and
- determine the clothing classification by inputting the subject image into the convolutional neural network, wherein the subject data comprises the clothing classification.

9. The medical system of any one of the preceding claims, wherein the instruction presentation system comprises any one of the following: a light amount control at the current station, a voice generation system at the current station, a room control at the current station, a video display system, an augmented reality device, a virtual reality device, and combinations thereof.

10. The medical system of any one of the preceding claims, wherein the instruction presentation system comprises a hand held computing device (116) and/or wherein the subject locator comprises the hand held computing device.

11. The medical system of any one of the preceding claims, wherein the set of stations comprises a preparation room (502) or preparation app and/or wherein the sequence of stations comprises an aftercare room (506) or aftercare app.

12. The medical system of claim 11, wherein the execution of the machine executable instructions is configured such that subject data collected at the preparation room or preparation app comprises any one of the following:
- a selection of a presentation theme for use by a subsequent station in the sequence of stations;
- physical mobility data collected in response to a physical ability questionnaire and/or in response to the presentation the at least one manual action, the at least one manual action comprising physical mobility test instructions;
- breathing data collected during the presentation of breath hold training instructions; and
- combinations thereof.

13. The medical system of any one of the preceding claims, wherein the subject data comprises subject sensor data.

14. The medical system of claim 13, wherein the subject measurement system comprises any one of the following for acquiring the subject sensor data: a wearable sensor, a station specific camera, a station specific microphone, a physiological sensor, a heart rate monitor, an accelerometer, a step counter, a respiratory sensor, and combinations thereof.

15. The medical system of any one of the preceding claims, wherein the medical system further comprises an operator interface (510) configured for enabling speech communication with the subject and/or for overriding the medical system, wherein overriding the medical system comprises any one of the following: halting the medical system, restarting the medial system, causing the medical system to repeat one or more of the set of stations for the subject, manually entering or editing the subject data, manually setting the current station, skipping the current station, skipping the subsequent station, and combinations thereof.

16. The medical system of any one of the preceding claims, wherein the instruction engine is a relational database or a look up table.

17. A method of operating a medical system (100),

18. Wherein the method comprises repeatedly controlling (200) a subject locator (136) for determining a subject presence at a current station of a set of stations (102), providing an indication of the current station, and to perform a verification procedure comprising:
- receiving (202) station specific instructions for the determined current station in response to inputting a received chosen protocol (144), the indication of the current station and an instruction mode (146) into an instruction engine (148), the station specific instructions comprising instructions for the subject to perform at least one manual action at the current station;
- presenting (204) the station specific instructions to the subject using an instruction presentation system (130, 132);
- controlling (206) a subject measurement (132, 322) system to measure subject data (134) in response to presenting the station specific instructions;
- assessing (208) a measure of completion (!52) of the at least one manual action using the subject data;
- modifying (212) the instruction mode if (210) the measure of completion meets a predetermined modification criterion (154);
wherein the method further comprises repeatedly performing the verification procedure, wherein in case the measure of completion meets (214) a predetermined completion criterion (156), the station specific instructions comprise an indication of a station subsequent (216) to the current station in accordance with a sequence of stations, wherein the chosen protocol specifies the sequence of stations.

19. A computer program comprising machine executable instructions (140) configured for controlling a computational system (106) to perform the method of claim 17.
